Europäisches Patentamt

European Patent Office

Office européen des brevets

(11) Veröffentlichungsnummer: **0 198 163**
**B1**

(12) EUROPÄISCHE PATENTSCHRIFT

(45) Veröffentlichungstag der Patentschrift:
26.07.89

(21) Anmeldenummer: 86101489.2

(22) Anmeldetag: 05.02.86

(51) Int. Cl.⁴: **A 61 F 2/30**

(54) Verbindung zweier Implantat-Teile.

(30) Priorität: 15.04.85 CH 1593/85

(43) Veröffentlichungstag der Anmeldung:
22.10.86 Patentblatt 86/43

(45) Bekanntmachung des Hinweises auf die Patenterteilung:
26.07.89 Patentblatt 89/30

(84) Benannte Vertragsstaaten:
AT DE FR GB IT

(56) Entgegenhaltungen:
EP-A- 0 000 549
EP-A- 0 121 491
CH-A- 541 963
DE-A- 2 548 077
DE-A- 2 839 093
DE-A- 3 138 848
FR-A- 1 017 927
FR-A- 2 391 711
US-A- 2 719 522

(73) Patentinhaber: GEBRÜDER SULZER
AKTIENGESELLSCHAFT, Zürcherstrasse 9,
CH-8401 Winterthur (CH)

(72) Erfinder: Frey, Otto, Wallrütistrasse 56,
CH-8400 Winterthur (CH)
Erfinder: Koch, Rudolf, Oberdorfstrasse 229,
CH-8267 Berlingen (CH)

(74) Vertreter: Dipl.-Ing. H. Marsch Dipl.-Ing. K. Sparing
Dipl.-Phys.Dr. W.H. Röhl Patentanwälte,
Rethelstrasse 123, D-4000 Düsseldorf 1 (DE)

## Beschreibung

Die Erfindung betrifft eine Verbindung zwischen zwei Implantat-Teilen, insbesondere für Gelenkendoprothesen, die im Verlauf der Implantierungsoperation miteinander so zu verbinden sind, daß Relativbewegungen zwischen ihnen vermieden sind, wobei der eine der beiden Teile einen Vater- und der andere einen Mutterkonus tragen, die eine gleiche Konizität haben, und wobei ferner gegen ein relatives Verdrehen beider Teile Sicherungselemente vorgesehen sind, die intraoperativ fixierbar und lösbar sind.

Um eine optimale Anpassung eines Implantates, beispielsweise einer Gelenkendoprothese, an den individuellen Patienten auch nur annähernd zu erreichen, ist es notwendig, eine Serie oder einen Bausatz des Implantats zur Verfügung zu halten, bei dem die unabhängigen «Variablen» – z.B. bei Knie- oder Hüftprothesen die Schaftlängen und/oder die Schaftdicken und zusätzlich bei Hüftgelenkprothesen die Prothesenhalslängen und/oder die Winkel zwischen der Schaftachse und der Prothesenhalsachse – beliebig miteinander kombiniert werden können. Das erfordert eine Vielzahl von Einzelprothesen für eine solche Serie, aus denen der Operateur intraoperativ die individuell günstigste auswählt.

Um diese Vielfalt zu verringern, ist es nützlich, das Implantat oder die Prothese aus mindestens zwei Teilen – beispielsweise einem Schaftteil mit variablen Längen und Dicken und einem Tibiaplateau unterschiedlicher Größe oder Dicke oder einem Halsteil mit unterschiedlichen Längen und Winkeln des Prothesenhalses – zusammenzustellen. Die Kombination der passenden Teile erfolgt dabei durch den Operateur intraoperativ, d.h. während der Implantation.

Dabei soll die Verbindung beider Teile wieder lösbar sein, um beispielsweise bei Reoperationen während einer späteren Operation den Schaft im Knochen zu belassen und nur den «funktionellen» Teil der Prothese, beispielsweise gegen einen, im allgemeinen größeren, auszutauschen, durch den einem gegebenenfalls weiter fortgeschrittenen Knochenabbau Rechnung getragen wird.

Hierbei besteht die Schwierigkeit, daß Relativbewegungen zwischen den beiden Teilen unter Belastungen vermieden werden müssen, die zu Abrieb, Lockerungen und Korrosionen führen können.

Aus der DE-A-3138848 ist daher bekannt, zwei Prothesenteile durch einen Konus lösbar miteinander zu verbinden und diese Verbindung durch Spannschrauben gegen unbeabsichtigtes Verdrehen zu sichern; weiterhin werden dort zylindrische Feinstgewinde als Verbindung zweier Implantatteile benutzt, wobei diese Verbindungen durch Sicherungsstifte gegen Verdrehen gesichert sind.

Aus der FR-A-1017927 ist weiterhin bekannt, zwischen zwei Endoprothesenteilen einen Gewindekonus als lösbare Verbindung anzuordnen. In der Praxis hat sich gezeigt, daß die von den relativ groben Gewinden dieser Konstruktion erzeugten Reibungskräfte unter Umständen nicht ausreichen, Relativbewegungen zwischen den Teilen völlig zu unterdrücken.

Es hat sich nun gezeigt, daß das «Einfädeln» eines mit einem Feinstgewinde versehenen Konus dem Operateur unter Umständen Schwierigkeiten bereitet, besonders bei Reoperationen, bei denen nur der «funktionelle» Teil der Prothese ausgewechselt wird. Hauptaufgabe der Erfindung ist es daher, dem Operateur den Zusammenbau zweier über Gewindekonen verbundener Implantatteile zu erleichtern; zusätzlich sollen bei der neuen Konstruktion der lösbaren Verbindung Relativbewegungen zwischen den beiden Implantatteilen mit Sicherheit verhindert werden. Diese Aufgabe wird mit der vorliegenden Erfindung dadurch gelöst, daß die Konizität zwischen 1 und 8% beträgt, daß weiterhin die Flächen der Konen ein Feinstgewinde mit einer Steigung von 0,1 bis 0,5 mm aufweisen, und daß schließlich an die Gewindekonen in Einsteckrichtung koaxial ein zylindrischer Führungsbereich anschließt, bei dem Bohrung und Zapfen als Schiebesitz aufeinander abgestimmt sind. Die «prozentuale Konizität» ist dabei definiert als das 100-fache der Differenz des großen und des kleinen Konusdurchmessers (D bzw. d) geteilt durch die Konuslänge L ( (D−d).100/L).

Durch den dem Konus vorgelagerten zylindrischen Führungsbereich wird das «Einfädeln» der beiden Gewindekonen entscheidend erleichtert; diese Wirkung beruht vor allem darauf, daß durch diesen Bereich die beiden Konen relativ zueinander exakt zentriert werden, womit ihr opimaler Sitz gewährleistet wird.

Der schlanke Konus bewirkt zwischen beiden Teilen eine Selbsthemmung, die in Verbindung mit dem Feinstgewinde auch unter Belastungen Relativbewegungen zwischen beiden Teilen verhindert. Das Feinstgewinde erleichtert dabei den festen «Anzug» des Konus und vergrößert die an diesem wirksamen Reibungskräfte.

Ein weiterer Vorteil des dem Konus vorgelagerten Führungsbereiches ist die Aufnahme von Biegebelastungen, gegen die die Flanken eines Feinstgewindes besonders anfällig sind.

Als vorteilhaft hat sich erwiesen, wenn die Sicherungselemente aus einem mit den Konen koaxialen Gegenkonus bestehen, in den ein Schraubenbolzen einschraubbar ist.

Um unabhängig von der Winkelstellung der beiden Implantat-Teile zueinander ein Festziehen der Verbindung zu ermöglichen, ist es zweckmäßig, wenn mindestens einer der beiden Teile rotationssymmetrisch bezüglich der Achse der Konen ausgebildet ist.

Im folgenden wird die Erfindung anhand von Ausführungsbeispielen im Zusammenhang mit der Zeichnung näher erläutert.

Fig. 1 ist schematisch ein Schnitt in Richtung der Längsachse der Verbindung;

Fig. 2 gibt vergrößert das Detail A aus Fig. 1 wieder;

Fig. 3 zeigt in gleicher Darstellung wie Fig. 1 und in einem Schnitt III-III von Fig. 4 eine Variante für ein lösbares Sicherungselement;

Fig. 4 ist der Schnitt IV-IV von Fig. 3;

Fig. 5 und 6 zeigen, ohne auf Details einzugehen, zwei Anwendungsbeispiele für die neue Verbindung.

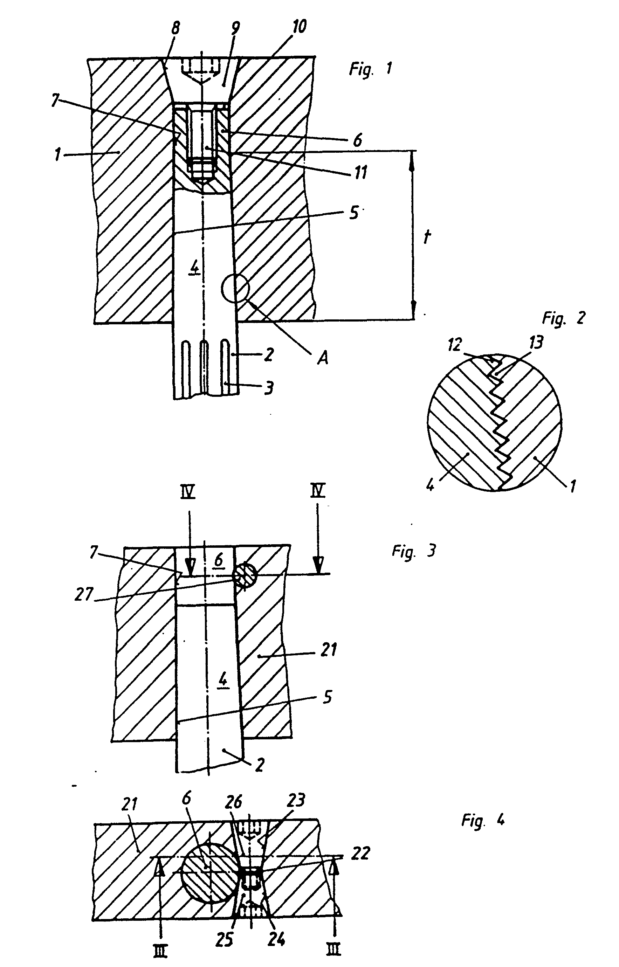

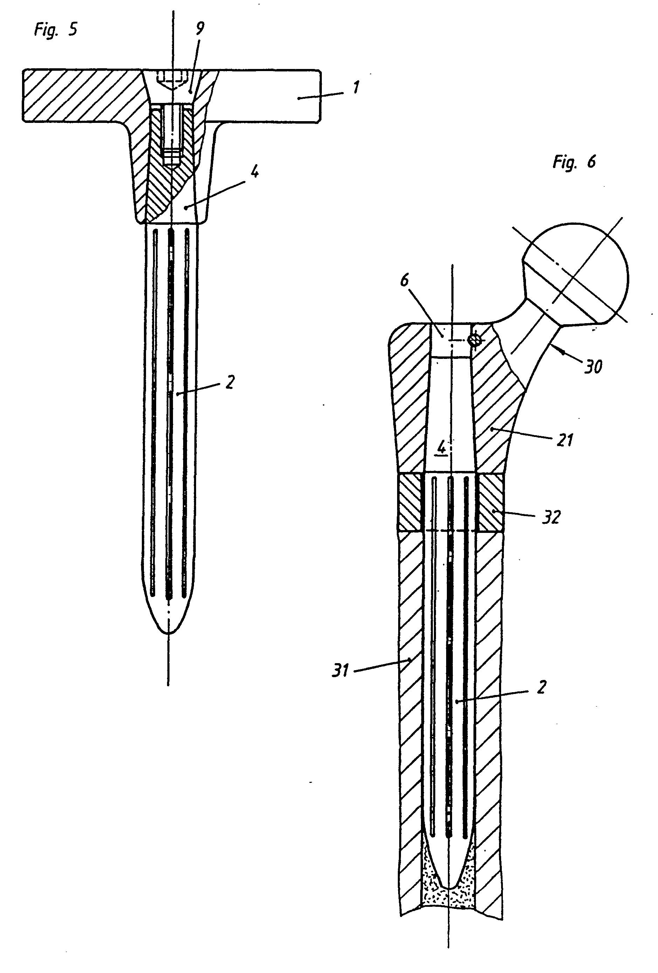

In Fig. 1 ist der eine Implantat-Teil, beispielsweise ein in Fig. 5 gezeigter Tibiateil 1 einer Kniegelenkprothese dargestellt, der mit Hilfe eines beispielsweise kreiszylindrischen Schaftes 2 im Schienbein verankert wird. Der Zylindermantel des Schaftes 2 ist mit parallel zu seiner Achse verlaufenden Verankerungsnuten 3 versehen.

An seinem proximalen Ende geht der Schaft 2 in einen Konus 4 über, der an einen entsprechenden Mutterkonus 5 im Tibiateil 1 angepasst ist. Beide Konen 4 und 5 reichen von distal bis zu einer Tiefe t in den Tibiateil 1 hinein und gehen dann in einen kreiszylindrischen Führungszapfen 6 bzw. eine entsprechende Bohrung 7 über, die als Schiebesitz aneinander angepasst sind, um eine Zentrierung der Konen beim «Einfädeln» zu erleichtern.

Während der Zapfen 6 das proximale Endstück des Schaftes 2 bildet, schließt an die Bohrung 7 des Tibiateils 1 ein sich in Gegenrichtung zu den Konen 4 und 5 öffnender Gegenkonus 8 an, der zur Aufnahme eines Sicherungsbolzens 9 dient. Dieser wird in eine in die Stirnfläche 10 des Zapfens 6 eingebrachte Gewindebohrung 11 eingeschraubt.

Wie Fig. 2 zeigt, sind die beiden Konen 4 und 5 – jedoch nicht der Zapfen 6 und die Bohrung 7 – mit je einem Feinstgewinde 12 bzw. 13 versehen, das beispielsweise eine Steigung von 0,2 mm hat.

In bekannter Weise ist dem Operateur die Möglichkeit gegeben, Tibiateil 1 und Schaft 2 aus einem Bausatz auf den Patienten hinsichtlich Grösse bzw. Länge und Dicke individuell abgestimmt auszuwählen, beide Teile intraoperativ über die konischen Gewinde 12 und 13 miteinander zu verschrauben und anschließend mit dem Sicherungsbolzen 9 zu sichern.

Die «Schlankheit» der Konen 4 und 5 und die geringe Steigung der Feinstgewinde 12, 13 gewährleisten dabei, daß ohne großen Kraftaufwand die Schraubverbindung derart angezogen werden kann, daß Relativbewegungen zwischen beiden Teilen 1 und 2 verhindert werden. Von Vorteil ist dabei, wenn der Schaftteil 2 bezüglich der Längsachse der Konen 4 und 5 rotationssymmetrisch ausgebildet ist, da dadurch ein stufenloses «Anziehen» der konischen Verschraubung ermöglicht wird.

Das Ausführungsbeispiel nach Fig. 3 und 4 unterscheidet sich von der ersten Ausführungsform der Erfindung nach Fig. 1 und 2 nur durch eine unterschiedliche Konstruktion des Sicherungselementes. Der eine zu verankernde Teil ist in diesem Fall beispielsweise ein blattartiger Halsteil 21 einer Femurkopfprothese 30, wie sie in Fig. 6 gezeigt ist. Der Halsteil 21 wird dabei wieder in einem kreiszylindrischen Schaft 2 verbunden. Im Bereich des Führungszapfens 6, der in diesem Fall mit einer von lateral nach medial verlaufenden Schulter (Fig. 6) des Halsteils 21 abschließt, ist durch den Halsteil 21 eine Durchgangsbohrung 22 vorgesehen, die einerseits geringfügig in einer Ausnehmung 27 des Führungszapfens 6 eingreift und andererseits von der Mitte der Halsteildicke aus nach beiden Seiten konisch erweitert. Diese Konen 23 und 24 dienen zur Aufnahme von einer mit entsprechenden konischen Köpfen versehenen Schraubensicherung, die aus einer Mutter 25 und einem Bolzen 26 besteht.

Bei dieser Konstruktion werden nach dem Verschrauben von Schaft 2 und Halsteil 21 die Sicherungselemente 25 und 26 in der Durchgangsbohrung 22 bzw. den Konen 23 und 24 montiert und miteinander verschraubt, wobei die Sicherung gegen unbeabsichtigtes Lösen der Konen 4 und 5 durch das Eingreifen der Elemente 25 und 26 in die Ausnehmung 27 sichergestellt wird.

Die mit der neuen Verbindung ausgestattete Femurkopf-Prothese 30 dient z.B. als Implantat für Patienten, bei denen als Folge eines Tumors eine umfangreiche Resektion des Femurknochens 31 erforderlich ist. Zur Einstellung der korrekten Beinlänge ist zwischen Knochen 31 und Halsteil 21 zusätzlich in bekannter Weise ein Distanzring 32 aus Kunststoff, vorzugsweise aus Polyäthylen, vorgesehen.

**Patentansprüche**

1. Verbindung zwischen zwei Implantat-Teilen (1, 2, 21), insbesondere für Gelenkendoprothesen, die im Verlauf der Implantierungsoperation miteinander so zu verbinden sind, daß Relativbewegungen zwischen ihnen vermieden sind, wobei der eine (2) der beiden Teile (1, 2, 21) einen Vater- (4) und der andere einen Mutterkonus (5) tragen, die gleiche Konizität haben, und wobei ferner gegen ein relatives Verdrehen beider Teile (1, 2, 21) Sicherungselemente (9; 25, 26) vorgesehen sind, die intraoperativ fixierbar und lösbar sind, dadurch gekennzeichnet, daß die Konizität zwischen 1 und 8 % beträgt, daß weiterhin die Flächen der Konen ein Feinstgewinde (12, 13) mit einer Steigung von 0,1 bis 0,5 mm aufweisen, und daß schließlich an die Gewindekonen (4, 5) in Einsteckrichtung koaxial ein zylindrischer Führungsbereich (6, 7) anschließt, bei dem Bohrung (7) und Zapfen (6) als Schiebesitz aufeinander abgestimmt sind.

2. Verbindung nach Anspruch 1, dadurch gekennzeichnet, daß die Sicherungselemente aus einem mit den Konen (4, 5) koaxialen Gegenkonus (8) bestehen, in den ein Schraubenbolzen (9) einschraubbar ist.

3. Verbindung nach Anspruch 1 oder 2, dadurch gekennzeichnet, daß mindestens einer (2) der beiden Teile (1, 2, 21) rotationssymmetrisch bezüglich der Achse der Konen (4, 5) ausgebildet ist.

**Claims**

1. A connection between two implant parts (1, 2, 21) more particularly for total joint endoprostheses, which during the implanting operation are required to be so interconnected that relative movements between them are obviated, one (2) of the two parts (1, 2, 21) being a male cone (4) and the other a female cone (5), which have identical conicity, and in which securing elements (9; 25, 26) are provided to prevent relative rotation of the two parts (1, 2, 21), such elements being intraoperatively fixable and releasable, characterised in that the conicity is between 1 and 8%, in that the surfaces of the cones have an extremely fine thread (12, 13) with a pitch of 0.1 to 0.5 mm, and finally in that the screwthreaded cones (4, 5) are followed coaxially in the direction of insertion by a cylindrical guide zone (6, 7) where the bore (7) and pin (6) are coordinated to form a sliding fit.

2. A connection according to claim 1, characterised in that the securing elements consist of a companion cone (8) coaxial with the cones (4, 5), a screw bolt (9) being screwable into said companion cone (8).

3. A connection according to claim 1 or 2, characterised in that at least one (2) of the two parts (1, 2, 21) is constructed to be rotationally symmetrical with respect to the axis of the cones (4, 5).

## Revendications

1. Liaison entre deux parties d'implant (1, 2, 21) notamment pour endoprothéses d'articulations, qui doivent être reliées l'une avec l'autre au cours de l'opération d'implantation de manière à empêcher des mouvements relatifs entre elles, l'une (2) des deux parties (1, 2, 21) portant un cône mâle (4) et l'autre portant un cône femelle (5) qui ont la même conicité, et il est en outre prévu pour empêcher une torsion relative des deux parties (1, 2, 21) des éléments de sûreté (9; 25, 26) qui peuvent être fixés et enlevés au cours de l'opération, caractérisée en ce que la conicité a une valeur entre 1 et 8% en ce qu'en outre, les faces des cônes présentent un filetage très fin (12, 13) avec un pas de 0,1 à 0,5 mm, et en ce que, finalement, une partie cylindrique de guidage (6, 7) dans laquelle un alésage (7) et un pivot (6) sont adaptés l'un à l'autre comme ajustement coulissant, se raccorde aux cônes filetés (4, 5) coaxialement dans la direction d'insertion.

2. Liaison suivant la revendication 1, caractérisée en ce que les éléments de sûreté sont constitués d'un contre-cône (8) coaxial aux cônes (4, 5), dans lequel un boulon (9) peut être vissé.

3. Liaison suivant la revendication 1 ou 2, caractérisée en ce qu'au moins l'une (2) des deux parties (1, 2, 21) est réalisée avec une symétrie de révolution par rapport à l'axe des cônes (4, 5).

Fig. 1

Fig. 2

Fig. 3

Fig. 4

Fig. 5

9

1

4

2

Fig. 6

6

30

21

4

32

31

2